# EUROPEAN PATENT SPECIFICATION

(11) **EP 0 803 246 B1**
(45) Date of publication and mention of the grant of the patent: **26.03.2003**
(21) Application number: 97201153.0
(22) Date of filing: 19.04.1997
(51) Int. Cl.: A61K 7/48

(54) **Compositions for topical application based on thermal muds and gelling agents**
Zusammensetzungen für topische Anwendung auf der Basis von Thermalschlamm und Geliermitteln
Compositions pour application topique à base de boues thermales et d'agents gélifiants

(30) Priority: 24.04.1996 CH 103696
(43) Date of publication of application: 29.10.1997
(73) Proprietor: APR APPLIED PHARMA RESEARCH S.A., 6855 Stabio (CH)
(72) Inventor: Reiner, Alberto, 22100 Como (IT); Reiner, Giorgio, 22100 Como (IT)
(74) Representative: Dragotti, Gianfranco

(56) References cited:
- EP-A- 0 646 647
- FR-A- 1 453 967
- FR-A- 2 498 451
- FR-A- 2 729 568
- GB-A- 695 916

## Description

The present invention relates to gelled compositions for cosmetic and/or pharmaceutical use based on more or less hydrated thermal muds; it also relates to the process for the preparation thereof. The beneficial effects, both of cosmetic and therapeutic nature, deriving from treatments and cures based on thermal muds have been well known from ancient times and form the basis of so∼called mud balneotherapy: it is also now universally recognised that thermal muds provide beneficial effects by means of a number of different actions, which can generally be summarised as thermal. mechanical and chemical stresses. EP-646647, GB-695916 and FR-1453967 disclose thermal mud compositions containing a gelling agents.

As regards thermal stresses, it has been found that mud, owing to its particular structure and granulometry and depending on the degree of hydration, is capable of absorbing substantial amounts of heat which can then be delivered slowly to the desired region of application; this feature differentiates mud from any other heat~transmitting means of therapeutic interest. In addition, owing to its plasticity, mud is capable of being shaped perfectly to the application surface and of being accepted by the latter without unpleasant side effects. For these reasons, thermal muds can also reach elevated temperatures of up to approximately 45°C without causing any thermal discomfort in the patient

However, as regards mechanical stresses it is important to underline the fact that, during treatment with thermal muds, a distinct reduction in intravascular pressure occurs in the human body, which facilitates both venous return and lymphatic drainage and also reduces stasis of interstitial liquids, with a consequent substantial improvement in venous circulation.

Finally, chemical stresses brought about by mud balneotherapy depend on the quantity and quality of the salts and gases dissolved in the waters accompanying thermal muds (the so~called thermal waters), although a further element of not inconsiderable importance is represented by any radiation contained therein. All thermal waters have a more or less variable content of the hydrosulphide anion (HS^{∼}) and they are therefore generally called sulphur waters, and they differ from one another further as a function of the other dissolved anions (for example, sulphurous/sulphate, sulphur-ous/bicarbonate. sulphurous/sulphate/bicarbonate, sulphurous bromoiodic waters, etc.).

Analogously, a further distinction can be made on the basis of the different cations present, among which there have been mentioned those of the alkali and alkaline earth metals, of iron and of arsenic.

However, as regards the dissolved gases, the capillary dilatation action brought about by hydrogen sulphate (H₂S) and carbon dioxide (CO₂) is of enormous importance. However, thermal mud treatments have the disadvantage of requiring that they be carried out in the area of the sources of mud, so that it has long been known that it is necessary to render thermal mud treatment possible near the users.

The present invention relates to novel gelled compositions for topical application, for cosmetic and/or therapeutic use, containing more or less hydrated thermal muds and at least one galling agent. The gelling agents may be either of natural origin, such as carrageenin, alginates, dextrins, dextrans, gelatins and xanthan gum, or of synthetic origin, such as carboxypolymethylene (Carbopol). carboxycelluloses (especially sodium carboxymethykellulose). hydroxycelluloses (especially hydroxypropylcellulose and hydroxymethylcellulose). acrylates and alkyl acrylates (especially mixtures of cross∼linked crylate and C_{10∼30} alkyl acrylate copolymers).

For the implementation of the present invention, it is preferable to use gelling substances of natural origin, such as the carrageenins normally available on the market.

It is even more preferable to use carrageenins purified in accordance with patent USA 5,246,702 which have a substantially lower molecular weight than have commercial carrageenins (approximately 10 times lower) and which, therefore, do not have the unpleasant disadvantage of leaving a kind of covering film on the epidermis so treated.

It has been found that carrageenin is capable of dispersing thermal muds homogeneously and of forming a micro-suspension therewith which can be distributed in an excellent and uniform manner on the epidermis. The gelled micro-suspension can readily be heated to a temperature of 38°C, fully preserving the dispersion of the mud and thus permitting excellent distribution on those parts of the body where intervention is necessary.

The gelled compositions according to the present invention, in addition to offering the indisputable cosmetic and therapeutic benefits of thermal muds, facilitating the methods of use, transport and dosage thereof, are also characterised by the ability to impart to the epidermis a particular and pleasant feeling of freshness, combined with a softness and cleanliness from all imperfections which may be found on the epidermis.

For that reason, a second subject of the present invention is constituted by the above∼mentioned gelled compositions based on thermal muds as true vehicles of active ingredients of varying nature dispersed or solubilised therein. The activity of some active ingredients for either cosmetic or therapeutic use is enhanced by the gelled mud and by the intrinsic properties which this mud thus vehicled already possesses.

The results obtained by using such vehicles for the active ingredients are better than those obtainable by using different vehicles for the active ingredients alone, especially as regards the cosmetic field. Therefore, by using the gelled muds, a series of finished cosmetic products has been prepared which range from face packs, individualised in accordance with the various skin types (distinguishing, for example, those characterised by blackheads or forms of acne from those requiring stimulation and functional renewal of the tissue), to anti~cellulite compositions.

It has also been found that the gelled compositions forming the subject~ matter of the present invention, irrespective of whether they contain additional active ingredients or not, may require slight heating, before the relevant application, to a temperature of approximately 38°C.

Before it can be used for the preparation of the gelled compositions forming the subject~matter of the present invention, the thermal mud has to undergo a pretreatment in accordance with the following general scheme.
1. Determination of the aqueous content of the mud mass which may vary from 20 to 80% by weight, depending on the thermal source of origin.
2. Mechanical sieving through a network of meshes having a mesh size of preferably not greater than 0.8 mm, and elimination of all material which has not been able to pass through the sieve.
3. Sterilisation of the filtrate; the sterilisation can be carried out using the normal standard techniques, preferably by using gamma rays of at least 25 KGy or by sterilisation in an autoclave in a current of steam.

Once the pretreatment has been carried out, the mud is heated to a temperature of from 25 to 90°C, preferably 60°C, after which the gelling substance, preferably carrageenin. is added. The amount of gelling substance which has to be added to obtain the compositions of the present invention is calculated on the basis of the amount of dry mud; to be more precise, this amount must be from 0.5 to 6% by weight relative to the amount of dry mud, preferably from 2 to 3.5% in the case of carrageenin and. even more preferably, 2.7%.

Analogously, if other gelling agents are used. these may be used alone or in combination with one another, in accordance with the percentages indicated below:

| | |
|---|---|
| • Cross~linked acrylate/C10∼30 alkyl acrylate copolymers: | 0.5∼3% |
| • Hydroxyethylcellulose: | 0.5∼3% |
| • Hydroxypropylcellulose: | 0.5∼3% |
| • Sodium carboxymethylcellulose: | 0.5∼4% |
| • Xanthan gum: | 0.5∼6% |
| • Alginates: | 0.2∼3% |
| • Dextrins: | 0.2∼3% |
| • Dextrans: | 0.2∼3% |
| • Gelatins: | 0.2∼6% |
| • Carboxypolymethylene (Carbopol): | 0.2∼3% |
| • Hydroxypropyl∼, hydroxyethyl∼, hydroxymethyl∼cellulose: | 0.5∼3% |

The mixture so obtained in left under agitation for a period of from 1 to 6 hours, preferably 2 hours, maintaining the temperature at from 25 to 90°C, preferably 60°C; the temperature is then adjusted to ambient values, generally 22°C, and the gelled composition is ready to be packaged.

Any additional active ingredients can be added during any phase of preparation, their thermal resistance being the only restriction.

The following are examples relating both to the process of preparation and to some particular types of gelled composition, and must be regarded as illustrative and non-limiting examples of the invention. In particular, both the active ingredients and the excipients used may vary in accordance with the requirements for which the finished product is intended.

### Example 1/Process of Preparation

After mechanical sieving using a network of meshes having a mesh size of 0.8 mm, 5000 g of mud having an aqueous content of 63% by weight were sterilised using gamma rays and then heated to a temperature of 60°C. 50 g of low~molecular~weight carrageenin purified in accordance with USA 5,246,702 were then added slowly and the mixture was subsequently left under agitation at a temperature of 65°C.

After 2 hours, the temperature was reduced to 22°C, a completely homogeneous gelled composition thus being obtained with a content of dry mud of 37%.

### Example 2/Mud Gell for Cellulite

| | |
|---|---|
| • 37% Gelled mud (dry residue): | 97.2 g |
| • Escine: | 0.4 g |
| • Mucopolysaccharide: | 0.3 g |
| • Deacetylated soya phospholipid: | 0.5 g |
| • Preservatives: | 1.6 g |

### Example 3/Mud gel for sunburn

| | |
|---|---|
| • 37% gelled mud (dry residue): | 94.9 g |
| • Allantoin: | 1.5 g |
| • Glycol extract of tomato* | 2 g |
| • Preservatives: | 1.6 g |

| | |
|---|---|
| * or equivalent amount corresponding to its dry extract. | |

### Example 4/Cream emulsion of gelled mud for skin imperfections

| | |
|---|---|
| 1) Active components: | |
| • Deacetylated soya phospholipid: | 0.5 g |
| • Mucopolysaccharide: | 0.3 g |
| • Hydroglycol extract of mallow* (GATTEFOSSE'): | 2 g |
| • 37% gelled mud (dry residue): | 50.5 g |
| 2) Lipid portion: | 14 g |
| 3) Water∼soluble and liposoluble preservatives: | 1.6 g |
| 4) Water: | balance to 100 g |
| 5) Perfume: | 0.75% |

| | |
|---|---|
| * or equivalent amount corresponding to its dry extract | |

### Example 5/Cream emulsion of gelled mud for cellulite

| 1) Active components: | |
|---|---|
| • Deacetylated soya phospholipid: | 0.5 g |
| • Mucopolysaccharide: | 0.3 g |
| • Escine: | 0.3 g |
| • Centella asiatica (GATTEFOSSE'): | 1 g |
| • Hydroglycol extract of horse chestnut* (GATTEFOSSE'): | 2 g |
| • Hydroglycol extract of seaweed* (GATTEFOSSE'): | 2 g |
| • 37% gelled mud (dry residue): | 50.5 g |
| 2) Lipid portion: | 14 g |
| 3) Water∼soluble and liposoluble preservatives: | 1.6 g |
| 4) Water: | balance to 100 g |
| 5) Perfume: | 0.75% |

| | |
|---|---|
| *or equivalent amount corresponding to its dry extract | |

### Example 6/Cream emulsion of gelled mud for the face

| 1) Active components: | |
|---|---|
| • Hydroglycol extract of mallow* (GATTEFOSSE') | 2 g |
| • Hydrolysed soya protein: | 5 g |
| • 37% gelled mud (dry residue): | 50.5 g |
| 2) Lipid portion: | 14 g |
| 3) Water∼soluble and liposoluble preservatives: | 1.6 g |
| 4) Water: | balance to 100 g |
| 5) Perfume: | 0.75% |

| | |
|---|---|
| *or equivalent amount corresponding to its dry extract. | |

### Example 7/Anti∼aging cream emulsion with vitamins of gelled mud

| | |
|---|---|
| 1) Active components: | |
| • Deacetylated soya phospholipids**: | 0.5 g |
| • Vitamin E acetate: | 0.75 g |
| • Vitamin C: | 0.75 g |
| • Hydroglycol extract of mallow* (GATTEFOSSE'): | 2 g |
| • Hydroglycol extract of arnica* (GATTEFOSSE'): | 2 g |
| • 37% gelled mud (dry residue): | 50.5 g |
| 2) Lipid portion: | 14 g |
| 3) Water∼soluble and lippsoluble preservatives: | 1.6 g |
| 4) Water: | balance to 100 g |
| 5) Perfume: | 0.75% |

| | |
|---|---|
| *or equivalent amount corresponding to its dry extract. | |
| **(L∼α∼glycerylphosphorylethanolamine/L∼α∼glycerylphosphoryl choline) | |

### Example 8/Cream emulsion for firming the bust based on gelled mud

| | |
|---|---|
| 1) Active components: | |
| • Deacetylated soya phospholipids** | 0.5 g |
| • Hydroglycol extract of hop*(GATTEFOSSE): | 2 g |
| • Hydroglycol extract of ginseng* (GATTEFOSSE'): | 2 g |
| • 37% gelled mud (dry residue): | 50.5 g |
| 2) Lipid portion: | 14 g |
| 3) Water∼soluble and liposoluble preservatives: | 1.6 g |
| 4) Water: | balance to 100 g |
| 5) Perfume: | 0.75% |

| | |
|---|---|
| *or equivalent amount corresponding to its dry extract. | |
| **(L∼α∼glycerylphosphorylethanolamine/L∼α∼glycerylphosphoryl choline) | |

### Example 9/Cream emulsion of gelled mud for the hair

| | |
|---|---|
| 1) Active components: | |
| • Deacetylated soya phospholipids** | 0.5 g |
| • Fresh plant amino∼protein complex: | 5 g |
| • Hydroglycol extract of nasturtium* (GATTEFOSSE): | 2 g |
| • 37% gelled mud (dry residue): | 50.5 g |
| 2) Upid portion: | 14 g |
| 3) Water∼soluble and liposoluble preservatives: | 1.6 g |
| 4) Water: | balance to 100 g |
| 5) Perfume: | 0.75% |

| | |
|---|---|
| *or equivalent amount corresponding to its dry extract. | |
| **(L∼α∼glycerylphosphorylethanolamine/L∼α∼glycerylphosphoryl choline) | |

### Example 10/Soothing face cream emulsion of gelled mud

| 1) Active components: | |
|---|---|
| • Deacetylated soya phospholipids** : | 0.5 g |
| • Hydroglycol extract of lime* (GATTEFOSSE'): | 2 g |
| • Hydroglycol extract of tomato* (GATTEFOSSE'): | 2 g |
| • Hydroglycol extract of aloe* (GATTEFOSSE'): | 2 g |
| • 37% gelled mud (dry residue): | 50.5 g |
| 2) Lipid portion: | 14 g |
| 3) Water∼soluble and liposoluble preservatives: | 1.6 g |
| 4) Water: | balance to 100 g |
| 5) Perfume: | 0.75% |

| | |
|---|---|
| *or equivalent amount corresponding to its dry extract. | |
| **(L∼α∼glycerylphosphorylethanolamine/L∼α∼glycerylphosphoryl choline) | |

### Example 11/Anti-stretch mark cream emulsion of gelled mud

| | |
|---|---|
| 1) Active components: | |
| • Deacetylated soya phospholipids**: | 0.5 g |
| • Hydroglycol extract of ivy* (GATTEFOSSE'): | 2 g |
| • Kydroglycol extract of papaya* (GATTEFOSSE'): | 2 g |
| g 37% gelled mud (dry residue): | 50.5 g |
| 2) Lipid portion: | 14 g |
| 3) Water~soluble and liposoluble preservatives: | 1.6 g |
| 4) Water: | balance to 100 g |
| 5) Perfume: | 0.75% |

| | |
|---|---|
| *or equivalent amount corresponding to its dry extract. | |
| **(L∼α∼glycerylphosphorylethanolamine/L∼α∼glycerylphosphoryl choline) | |

### Example 12/Anti∼acne cream emulsion of gelled mud

| | |
|---|---|
| 1) Active components: | |
| • Deacetylated soya phospholipids** | 0.5 g |
| • Ethyl azelate | 0.5 g |
| • Hydroglycol extract of lavender* (GATTEFOSSE'): | 2 g |
| • 37% gelled mud (dry residue): | 50.5 g |
| 2) Lipid portion: | 14 g |
| 3) Water∼soluble and liposoluble preservatives: | 1.6 g |
| 4) Water: | balance to 100 g |
| 5) Perfume: | 0.75% |

| | |
|---|---|
| *or equivalent amount corresponding to its dry extract. | |
| **(L∼α∼glycerylphosphorylethanolamine/L∼α∼glycerylphosphory choline). | |

## Claims

1. Compositions containing thermal mud with an aqueous content of from 20 to 80% by weight together with at least one gelling agent, **characterised in that** the gelling agent is carrageenin and it is present in an amount of from 0.5 to 6% by weight with respect to the dry mud.

2. Compositions according to Claim 1, **characterised in that** carrageenin is present in an amount of from 2 to 3.5% by weight with respect to the dry mud.

3. Compositions according to Claim 2, **characterised in that** carrageenin is present in an amount of 2.7% by weight with respect to the dry mud.

4. Compositions according to Claim 1, **characterised in that** carrageenin is purified carrageenin having a low molecular weight.

5. Compositions for cosmetic use according to any one of the preceding claims.

6. Compositions according to Claims 1-5, **characterised in that** they contain at least one active ingredient for topical application.

7. Compositions according to Claim 6, **characterised in that** said active ingredient for topical application is provided with cosmetic activity.

8. Compositions according to Claim 7, **characterised in that** said active ingredient provided with cosmetic activity is selected from: deacetylated soya phospholipids, mucopolysaccharides, allantoin, escine, Centella asiatica, vitamins, dry or plant hydroglycol extracts.

9. Compositions according to Claim 8, **characterised in that** the deacetylated soya phospholipids are selected from: L-α-glycerylphosphorylethanolamine and L-α-glycerylphosphoryl choline.

10. Process for the preparation of compositions according to Claim 1, **characterised by** the following stages:
a) mechanical sieving through a network of meshes,
b) sterilisation of the filtrate,
c) heating of the sterilised filtrate and addition of the at least one gelling agent, the gelling agent being carrageenin under stirring.

11. Process according to Claim 10, **characterised in that** the meshes of the mesh network are of a size not greater than 0.8 mm.

12. Process according to Claim 10, **characterised in that** the sterilisation is carried out by the use of gamma rays of at least 25 KGy or in an autoclave in a current of steam.

13. Process according to Claim 10, **characterised in that** the heating is carried out at a temperature of from 25 to 90°C.

14. Process according to Claim 13, **characterised in that** the heating is carried out at a temperature of 60°C.

15. Process according to Claim 10, **characterised in that** the gelling agent is added at a temperature of 60°C.

16. Process according to Claim 10, **characterised in that** the so-obtained mixture is maintained under stirring at a temperature of from 25 to 90°C for a period of from 1 to 6 hours.

17. Process according to Claim 16, **characterised in that** stirring is maintained for a period of 2 hours.

18. Process according to Claim 16, **characterised in that** stirring is carried out at a temperature of 60°C.

## Patentansprüche

1. Zusammensetzungen, enthaltend Thermalschlamm mit einem Wassergehalt von 20 bis 80 Gew.% zusammen mit mindestens einem Geliermittel, **dadurch gekennzeichnet, dass** das Geliermittel Karrageen ist und in einer Menge von 0,5 bis 6 Gew.% im Hinblick auf den trockenen Schlamm vorliegt.

2. Zusammensetzungen gemäss Anspruch 1, **dadurch gekennzeichnet, dass** das Karrageen in einer Menge von 2 bis 3,5 Gew.% im Hinblick auf den trockenen Schlamm vorliegt.

3. Zusammensetzungen gemäss Anspruch 2, **dadurch gekennzeichnet, dass** das Karrageen in einer Menge von 2,7 Gew.% im Hinblick auf den trockenen Schlamm vorliegt.

4. Zusammensetzungen gemäss Anspruch 1, **dadurch gekennzeichnet, dass** das Karrageen gereinigtes Karrageen mit einem niedrigen Molekulargewicht ist.

5. Zusammensetzungen zur kosmetischen Verwendung gemäss einem der vorstehenden Ansprüche.

6. Zusammensetzungen gemäss Ansprüchen 1 bis 5, **dadurch gekennzeichnet, dass** sie mindestens einen aktiven Bestandteil für die topische Anwendung enthalten.

7. Zusammensetzungen gemäss Anspruch 6, **dadurch gekennzeichnet, dass** der aktive Bestandteil für die topische Anwendung mit kosmetischer Aktivität versehen ist.

8. Zusammensetzungen gemäss Anspruch 7, **dadurch gekennzeichnet, dass** der aktive Bestandteil, versehen mit kosmetischer Aktivität, ausgewählt ist aus: deacetylierten Sojaphospholipiden, Mucopolysacchariden, Allantoin, Escin, Centella asiatica, Vitaminen, trockenen oder Pflanzenhydroglykolextrakten.

9. Zusammensetzungen gemäss Anspruch 8, **dadurch gekennzeichnet, dass** die deacetylierten Sojaphospholipide gewählt werden aus: L-α-Glycerylphosphorylethanolamin und L-α-Glycerylphosphorylcholin.

10. Verfahren für die Herstellung von Zusammensetzungen gemäss Anspruch 1, **gekennzeichnet durch** die folgenden Schritte:
(a) mechanisches Sieben **durch** ein Netzwerk von Maschen,
(b) Sterilisierung des Filtrats,
(c) Erwärmen des sterilisierten Filtrats und Zugabe von dem mindestens einen Geliermittel unter Rühren, wobei das Geliermittel Karrageen ist.

11. Verfahren gemäss Anspruch 10, **dadurch gekennzeichnet, dass** die Maschen des Maschennetzwerks eine Grösse von nicht mehr als 0,8 m aufweisen.

12. Verfahren gemäss Anspruch 10, **dadurch gekennzeichnet, dass** die Sterilisation unter Verwendung von γ-Strahlen von mindestens 25 KGy oder in einem Autoklaven in einem Dampfstrom durchgeführt wird.

13. Verfahren gemäss Anspruch 10, **dadurch gekennzeichnet, dass** die Erwärmung bei einer Temperatur von 25 bis 90°C durchgeführt wird.

14. Verfahren gemäss Anspruch 13, **dadurch gekennzeichnet, dass** die Erwärmung bei einer Temperatur von 60°C durchgeführt wird.

15. Verfahren gemäss Anspruch 10, **dadurch gekennzeichnet, dass** das Geliermittel bei einer Temperatur von 60°C zugefügt wird.

16. Verfahren gemäss Anspruch 10, **dadurch gekennzeichnet, dass** die so erhaltene Mischung unter Rühren bei einer Temperatur von 25 bis 90°C für eine Zeitspanne von 1 bis 6 Stunden gehalten wird.

17. Verfahren gemäss Anspruch 16, **dadurch gekennzeichnet, dass** das Rühren für eine Zeitspanne von 2 Stunden aufrechterhalten wird.

18. Verfahren gemäss Anspruch 16, **dadurch gekennzeichnet, dass** das Rühren bei einer Temperatur von 60°C durchgeführt wird.

## Revendications

1. Compositions contenant une boue thermale avec une teneur aqueuse comprise entre 20 et 80 % en poids en mélange avec au moins un gélifiant, **caractérisées en ce que** le gélifiant est la carraghénine et qu'il est présent en quantité comprise entre 0,5 et 6 % en poids par rapport à la boue sèche.

2. Compositions selon la revendication 1, **caractérisées en ce que** la carraghénine est présente en quantité comprise entre 2 et 3,5 % en poids par rapport à la boue sèche.

3. Compositions selon la revendication 2, **caractérisées en ce que** la carraghénine est présente en quantité de 2,7 % en poids par rapport à la boue sèche.

4. Compositions selon la revendication 1, **caractérisées en ce que** la carraghénine est de la carraghénine purifiée ayant un faible poids moléculaire.

5. Compositions pour utilisation cosmétique selon l'une quelconque des revendications précédentes.

6. Compositions selon les revendications 1 à 5, **caractérisées en ce qu'**elles contiennent au moins un ingrédient actif pour application topique.

7. Compositions selon la revendication 6, **caractérisées en ce que** ledit ingrédient actif pour application topique est fourni avec une activité cosmétique.

8. Compositions selon la revendication 7, **caractérisées en ce que** ledit ingrédient actif fourni avec une activité cosmétique est choisi parmi : des phospholipides de soja désacétylé, des mucopolysaccharides, l'allantoïne, l'escine, le Cantella asiatica, des vitamines, des extraits de plantes hydroglycoliques.

9. Compositions selon la revendication 8, **caractérisées en ce que** les phospholipides de soja désacétylé sont choisis parmi : la L-α-glycérylphosphoryl-éthanolamine et la L-α-glycérylphosphoryl-choline.

10. Procédé pour la préparation de compositions selon la revendication 1, **caractérisé par** les étapes suivantes :
a) tamisage mécanique à travers un réseau maillé,
b) stérilisation du filtrat
c) chauffage du filtrat stérilisé et addition du au moins un gélifiant, le gélifiant étant la carraghénine, sous agitation.

11. Procédé selon la revendication 10, **caractérisé en ce que** les mailles du réseau maillé sont de taille non supérieure à 0,8 mm.

12. Procédé selon la revendication 10, **caractérisé en ce que** la stérilisation est réalisée en utilisant des rayons gamma d'au moins 25 KGy ou dans une autoclave sous un courant de vapeur.

13. Procédé selon la revendication 10, **caractérisé en ce que** le chauffage est mené à une température comprise entre 25 et 90 °C.

14. Procédé selon la revendication 13, **caractérisé en ce que** le chauffage est mené à une température de 60 °C.

15. Procédé selon la revendication 10, **caractérisé en ce que** le gélifiant est ajouté à une température de 60 °C.

16. Procédé selon la revendication 10, **caractérisé en ce que** le mélange ainsi obtenu est maintenu sous agitation à une température comprise entre 25 et 90 °C pendant une durée allant de 1 à 6 heures.

17. Procédé selon la revendication 16, **caractérisé en ce que** l'agitation est maintenue pendant une durée de 2 heures.

18. Procédé selon la revendication 16, **caractérisé en ce que** l'agitation est effectuée à une température de 60 °C.
